# EUROPEAN PATENT APPLICATION

(11) **EP 0 044 140 A1**
(43) Date of publication of application: **20.01.1982**
(21) Application number: 81302668.9
(22) Date of filing: 15.06.1981
(51) Int. Cl.: G01N 33/54, G01N 33/76, G01N 33/78, G01N 33/94, G01N 33/74, G01N 33/82

(54) **Binding assays**

(30) Priority: 13.06.1980 GB 8019430
(71) Applicant: NATIONAL RESEARCH DEVELOPMENT CORPORATION, London SE1 6BU (GB)
(72) Inventor: Collins, William Patrick, Worcester Park Surrey (GB); Barnard, Geoffrey John Russel, Sutton Surrey (GB); Matson, Christine Mary, (Née Branch), East Dulwich SE 22 (GB)
(74) Representative: Stephenson, Gerald Frederick (GB)

(57) **Abstract**

A method of carrying out binding assay (e.g. immunoassay) comprises treating the aqueous reaction mixture, subsequent to the binding interaction, with a reactant (e.g. an enzyme) which interacts preferentially with a labelled version of one or other of the binding reagents when said reagent is either bound in a complex or more usually in the free state, to give rise to a product having solubility properties which are substantially different from those of the original binding reagent or complex, thereafter partitioning hydrophobic substances from the treated reaction mixture into a hydrophobic solvent phase and detecting or determining labelled material present in the hydrophobic phase. The method, which is particularly applicable to the immunoassay of hormones and drugs and their hydrophilic metabolite conjugates, relies upon the differential accessibility to reaction of one or other of the binding reagents when in the free state as compared with the bound state and utilises this to selectively alter the solubility properties of either bound or free material to facilitate a liquid-liquid partition to separate labelled free and bound material. This liquid-liquid separation replaces the solid phase separation step of previous assays advantageously providing a pseudo-homogeneous binding assay. Also the partition separation advantageously separates labelled material from luminescently or fluorescently-active contaminants contained in the aqueous sample and is thus desirable for use with luminescent and fluorescent labelled immunoassay procedures.

## Description

This invention relates to binding assays, such as receptor assays and immunoassays, including in particular radioimmunoassay (RIA), luminescence-labelled immunoassay (LIA) and fluorescence- labelled immunoassay (FIA).

In recent years immunoassay and other binding assay techniques have been very widely adopted for measuring the concentration of biologically active substances such as antibodies and antigens, e.g. hormones and drugs and their metabolites, at the very low concentrations at which these substances are present and active in biological systems. Binding assay techniques rely upon a binding reaction of the substance undergoing assay with a corresponding binding partner which has a specific binding affinity for the substance in preference to other substances; for instance a specific antigen-antibody binding reaction. The binding reaction is monitored by use of a suitable label on one or other of the reagents, the proportion of labelled material present in either the free or bound state after incubation being measured. In order to carry out this measurement it is generally necessary physically to separate free labelled reagent from bound complexed labelled reagent. Thus, for example, in a radioimmunoassay technique for determination of a hormone, a sample containing an unknown quantity of the hormone is incubated, together with a known quantity of a radioactively labelled analogue of the hormone, with a limited fixed quantity of a specific antibody to the hormone; the labelled and unlabelled hormone is thereby competing for binding sites on the limited quantity of antibody. After incubation the bound hormone- antibody complex, which may be in the solid phase, is separated from the free hormone, e.g. by filtration, the radioactive label is measured as a component of either the free or bound material and the quantity of unknown hormone originally present in the sample is thereby determined. The solid phase separation step, which is an essential characteristic of conventional radioimmunoassay and other heterogeneous immunoassay techniques, is an undesirable manipulative step which complicates the procedure rendering it difficult to automate.

Recently homogeneous immunoassay methods have been proposed, typically relying upon a change in some property of the label when in the bound state as compared with the free state. This change in properties, for example a change in reactivity or luminescence or fluoresecence properties, is used to determine the extent of binding and thus the quantity of the unknown. For example, U.K. Patent Specification No. 1,552,607 (Miles Laboratories Inc.) describes very broadly defined homogeneous methods of carrying out specific binding assays utilising, in particular, luminescence and fluorescence measurements. Also, it has been proposed in published U.K. Patent Application No. 2008247 (The Welsh National School of Medicine) to carry out a homogeneous immunoassay in which a luminescent-labelled substance is reacted with a fluorescently labelled antigen or antibody therefor, and a luminescent reaction is triggered, the energy of the luminescent reaction interacting with and exciting the fluorescent label on the bound antigen or antibody to produce a wavelength shift in light emission or a change in quantum yield. Biological samples, such as those derived from blood or urine, however, inevitably contain extraneous substances which may interfere with luminescence and fluorescence measurements, for instance, by production of background radiation against which it is extremely difficult to determine the radiation due to the label only, let alone any shift in wavelength or change in quantum yield on binding.

A generally applicable method of carrying out binding assays has now been devised which advantageously obviates the need for a solid phase separation step, and is thereby more readily automated than previous methods incorporating such a step. The new method may conveniently also assist, in fluorescence and luminescence binding assays, by diminshing problems of interference due to extraneous substances present in biological samples.

Accordingly the present invention comprises a method of carrying out a binding assay comprising treating the aqueous reaction mixture, subsequent to the binding interaction, with a reactant which interacts preferentially with a labelled version of one or other of the binding reagents, when said reagent is either in the free state or bound in a complex, to give rise to a product having solubility properties which are substantially different from those of the original binding reagent or complex, thereafter partitioning hydrophobic substance from the treated aqueous reaction mixture into a hydrophobic solvent phase and detecting or determining labelled material present in the hydrophobic phase.

Thus the method of the present invention relies upon the differential accessibility to reaction of one or other of the binding reagents, e.g. antigen or antibody, when in the free state as compared with the bound complexed state and utilises this differential accessibility to alter selectively the solubility properties of either bound or free material to facilitate thereby a liquid-liquid partition separation of labelled free or bound material from the aqueous reaction mixture into a hydrophobic phase in which labelled material is then determined. This liquid-liquid separation replaces the solid phase separation step used in previous binding assay techniques, such as RIA. Consequently the binding assays of the invention may be carried out completely in the liquid phase and as such may be viewed as pseudo-homogeneous binding assays.

The method of the present invention is widely applicable to binding assays in general in which a substance is detected or determined by way of a specific binding reaction of that substance, with a binding partner therefor which has a specific binding affinity for that substance in preference to other substances. Thus the method may be applied to all those specific binding assay procedures which have been used previously, such as protein binding assays, receptor assays and specific enzyme assays, and especially immunoassays. The substance undergoing assay may be any of those substances for which a suitable binding partner can be found, including peptides, proteins, carbohydrates, glyco-proteins, nucleotides, bile acids, steroids and prostanoids. In particular, the method may be used in the binding assay of antigens or haptens and their corresponding antibodies, for instance, for binding assay of hormones, vitamins, pharmacological agents, drugs and metabolites and binding substances and receptors of any of these. Specific examples of substances which may be assayed are hormones and drugs and derivatives of these, such as hydrophilic metabolite conjugates, e.g. sulphate or glucuronide drug or hormone conjugates.

Any suitable labelling tracer may be used to monitor the binding reaction, including those labelling tracers which are customarily used in binding assay techniques. Thus the label may comprise an enzyme, co-factor or enzyme inhibitor label, or a metal, dye, bacterial or viral label. In particular, the method of the invention is applicable to binding assays utilising radionuclide labelling including gamma-emitting radionuclide labels, e.g. Se and 1251 labels, and especially beta-emitting radionuclide labels such as tritium. When the radionuclide label is such a beta-emitting label, the hydrophobic phase into which labelled hydrophobic material is partitioned conveniently comprises the scintillation fluid for scintillation counting of the radioactive label. Preferably, also, luminescent and fluorescent labelling may be used, in which case partition of labelled material into the hydrophobic phase advantageously separates the labelled material from the aqueous sample and any luminescently or fluorescently active contaminants which it may contain. Furthermore, measurement of luminescent labels in the hydrophobic phase may give rise to enhancement of sensitivity (e.g. quantum yield), for instance, permitting use of peroxyoxalate chemiluminescence techniques. Examples of luminescent labels which may be used are acridines, isoluminol derivatives and dioxetanes, and examples of fluorescent labels are fluorescein labels, e.g. fluorescein isothiocyanate.

The binding interaction between the substance undergoing assay and its binding partner, e.g. antigen-antibody interaction, which is characteristically an aqueous phase interaction, may comprise any appropriate binding essay procedure. Thus the binding interaction may comprise a direct binding technique or a competitive binding technique, including both displacement binding and sequential saturation techniques.

Subsequent to the binding interaction the aqueous reaction mixture is treated with a reactant which interacts with the labelled version of one or other of the binding reagents, i.e. the substance or associated group of substances undergoing assay, or the specific binding partner thereof. The reactant preferentially interacts with the binding reagent when it is either bound in a complex or when it is in the free state, giving rise to a product the nature of which is characteristically such that it is possible to separate the resultant free or bound, complexed material from the aqueous reaction mixture by solvent partition procedures. Thus the present invention envisages four possible schemes:
A. in which the reactant preferentially interacts with reagent when in the form of a hydrophobic complex giving rise to a hydrophilic complex product enabling separation of free hydrophobic materials from the resultant reaction mixture by solvent partition procedures;
B. in which the reactant preferentially interacts with reagent when in the form of a hydrophilic complex giving rise to a hydrophobic complex product which may be separated from the resultant reaction mixture by solvent partition procedures;
C. in which reactant interacts preferentially with free hydrophobic reagent giving rise to a free hydrophilic product enabling separation of hydrophobic complex from the resultant reaction mixture by solvent partition procedures, and
D. in which reactant interacts preferentially with free hydrophilic reagent giving rise to a free hydrophobic product which may be separated from the resultant reaction mixture by solvent partition procedures.

The invention envisages that the reagent may be more accessible to the reactant when in the bound complexed state, as in A and B above,for instance,when the binding partner is, or is complexed with, a co-factor which enhances the reactivity of the reactant. More usually, however, the reactant interacts preferentially with the reagent when it is in the free state, as formation of a complex between the reagent and its binding partner normally inhibits reagent-reactant interaction. Advantageously the preferential interaction of the reactant is such that differentiation between the free and bound reagent is substantially maximised, the reactant preferably interacting with the reagent substantially only when it is in one state, usually the free state, and negligibly with the reagent when in its other state, usually the bound state.

The product formed on interaction of the reactant with the reagent may comprise a fragment of the original reagent cleaved therefrom by the action of the reactant. Such a fragment may consist of the label itself, e.g. when the label comprises a luminescent label such as an acridine ester or isoluminal substituent, or more usually the fragment comprises a grouping containing the label, e.g. when the label comprises a tritiated carboxylate or amino acid substituent. Thus, for example, the method may be used for detection and determination of water soluble hydrophilic materials, in particular high molecular weight materials such as proteins, in which case a reagent of the binding assay may comprise an analogue of the hydrophilic material comprising a labelled prosthetic group all or part of which may be cleaved from the hydrophilic material to yield a hydrophobic labelled fragment. It will be appreciated that reactants which interact with such analogue reagents will not usually interact with the native compound, e.g. protein, which does not contain the prosthetic group. Examples of suitable analogues are labelled, e.g. tritiated carboxylate or amino acid, e.g. propionate or tryptophan, conjugates of the native hydrophilic material which on interaction with a suitable reactant such as an enzyme, e.g.,8 -chymotrypsin or tryptophanase, release the corresponding labelled hydrophobic acid or portion thereof, e.g. propionic acid or indole (i.e. portion of tryptophan).

Alternatively the product may comprise the original reagent, in bound or free form, altered on interaction of the reactant by addition or removal of a functional grouping, e.g. an ester grouping or sugar residue, by means of which the hydrophilic/ hydrophobic solubility properties of the reagent are changed. It will be appreciated that in this case the reactant will interact with labelled and unlabelled material alike, including the substance undergoing assay, though the performance of the method of the invention relies only upon interaction with labelled material to differentiate between free and bound labelled material.

Thus in one embodiment the invention comprises a method of carrying out a binding assay for a hydrophilic substance, such as a water-soluble derivative of a hormone or drug, e.g. a sulphate or glucuronide ester, or sugar conjugate of a drug or hormone, in which, subsequent to the binding intereaction, the aqueous reaction mixture is treated with a reactant which interacts preferentially with a labelled version of the hydrophilic substance when either in the free state or when bound in a complex, usually when in the free state, to give rise to a labelled hydrophobic product, e.g. by hydrolysis of the hydrophilic derivative,which is thereafter partitioned from the treated aqueous reaction mixture and determined.

Alternatively in a further embodiment the invention comprises a binding assay for a substance which is a relatively hydrophobic substance, such as a drug or a hormone, which comprises treating the aqueous reaction mixture, subsequent to the binding interaction, with a reactant which interacts preferentially with a labelled version of the substance, either when in the free state or when bound in a complex, usually when in the free state, to give rise to a hydrophilic product, e.g. by transformation of the labelled material into a corresponding hydrophilic derivative such as an ester or sugar conjugate, and thereafter partitioning hydrophobic bound complex comprising labelled material from the treated reaction mixture into a hydrophobic phase and determining said labelled material.

Any suitable reactant and treatment may be used to derive the labelled product from the labelled reagent. Such treatment may include mild chemical treatment, for instance, with weakly acid or alkaline solution to hydrolyse hydrophilic ester groupings e.g. acridine ester groupings. Treatment may also comprise treatment with reactants which do not cause any apparent chemical transformation of the reagent. For example, it has been found in accordance with the present invention that treatment of a prostaglandin (PGFM) or a drug (e.g. digoxin) with saturated ammonium sulphate solution alters the solubility properties of these compounds rendering them hydrophobic and susceptible to separation from the reaction by partition into an organic solvent.

Usually, however, the reactant comprises an enzyme, in particular a specific conjugating enzyme or a specific esterase. For example, when the substance undergoing assay is a hydrophilic derivative, e.g. a water-soluble derivative of a hormone or drug, the reactant may comprise an esterase which hydrolyses the derivative to produce a hydrophobic parent compound, e.g. hydrolysis of a hydrophilic hormone glucuronide or sulphate by a glucuronidase or sulphatase to the corresponding hydrophobic hormone. Similarly, for example, when the substance is a water-soluble high molecular weight material such as a protein, the reactant may comprise an esterase to hydrolyse off a hydrophobic labelled prosthetic group, which may conveniently comprise a labelled, e.g. 3H labelled, ester grouping e.g. glucuronic acid ester group, and thus the reactant used may be the same as or similar to that used for assay of lower molecular weight hydrophilic derivatives. Furthermore, for example, when the substance undergoing assay comprises a relatively hydrophobic substance, such as a hormone, the reactant may comprise a specific conjugating enzyme together with a suitable derivative, e.g. UDP-glucuronyl transferase together with UDP-glucuronic acid.

Hydrophobic substances are then partitioned from the treated reaction mixture into a hydrophobic solvent. Any suitable solvent and conditions may be used for partition, and these may be varied in accordance with the relative solubility properties of the labelled product and the original binding reagent or complex. Conveniently the hydrophobic solvent phase may comprise the scintillation fluid for use in beta-emitting radionuclide labelled binding assays.

The binding assay method of the present invention may be carried out completely in the liquid phase, all reagents being added in the form of liquids, and thus the method is particularly suited to automation in comparison with prior art radioimmunoassay and other immunoassay methods which require a solid phase separation step. Conveniently, also, measurement of labelled material in the hydrophobic solvent phase overcomes problems of interference due to extraneous substances present in the original aqueous sample, which is particularly advantageous for fluorescence and luminescence immunoassays. Furthermore, in preferred embodiments the method of the invention may be advantageously based upon simple, natural, biological reactions, such as hydrolysis and esterification of hydrophilic derivates, e.g. glucuronide and sulphate derivatives.

The invention also includes kits for carrying out the method of the invention, typically comprising binding assay reagents together with a reactant, usually an enzyme, e.g. esterase or specific conjugating enzyme and derivative, for interacting with a labelled version of one or other of the binding reagents when said reagent is either in the free state or bound in a complex to give rise to a product having solubility properties which are substantially different from those of the original binding reagent or complex. The binding assay reagents usually comprise a labelled binding reagent, usually labelled antigen, e.g. labelled hormone, together with its corresponding binding partner, usually antibody, and reference material i.e. solutions or freeze-dried preparations containing known concentrations of the substance undergoing assay. When the kit comprises an enzyme reactant it may also comprise co-factor for the enzyme, and also substrate in the case of conjugating enzyme reactants. Antibody and labelled antigen may be maintained together in a single container, for instance in freeze-dried form. Kits may also comprise hydrophobic solvents, including scintillant fluids, for partitioning hydrophobic material from the aqueous reaction mixture.

Examples of some systems to which the liquid differentiation immunoassay procedure (LIDIA) of the present invention may be applied are given below in table form indicating the substance which may be assayed and labelled reagent, reactants and solvent partitions which may be used.

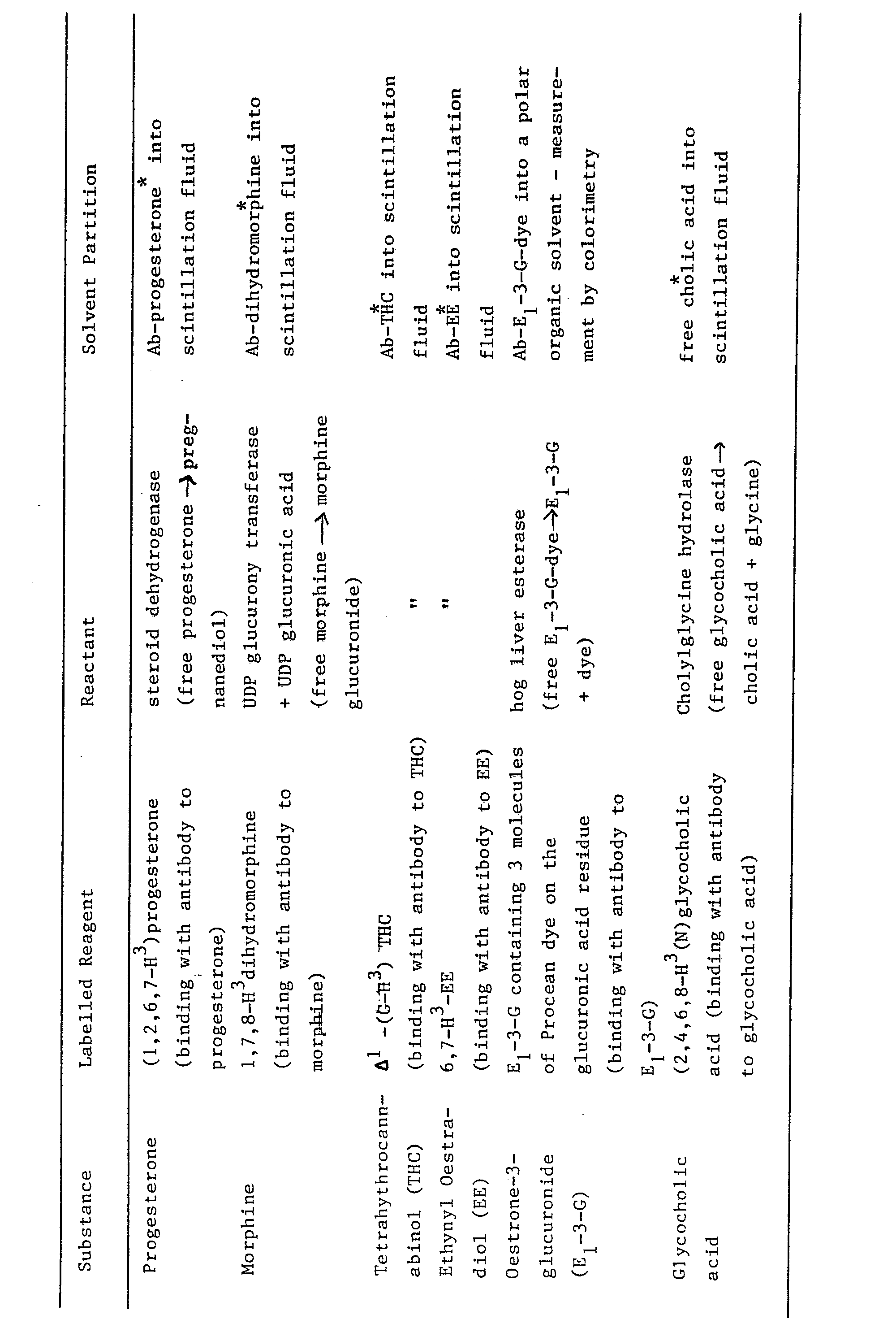

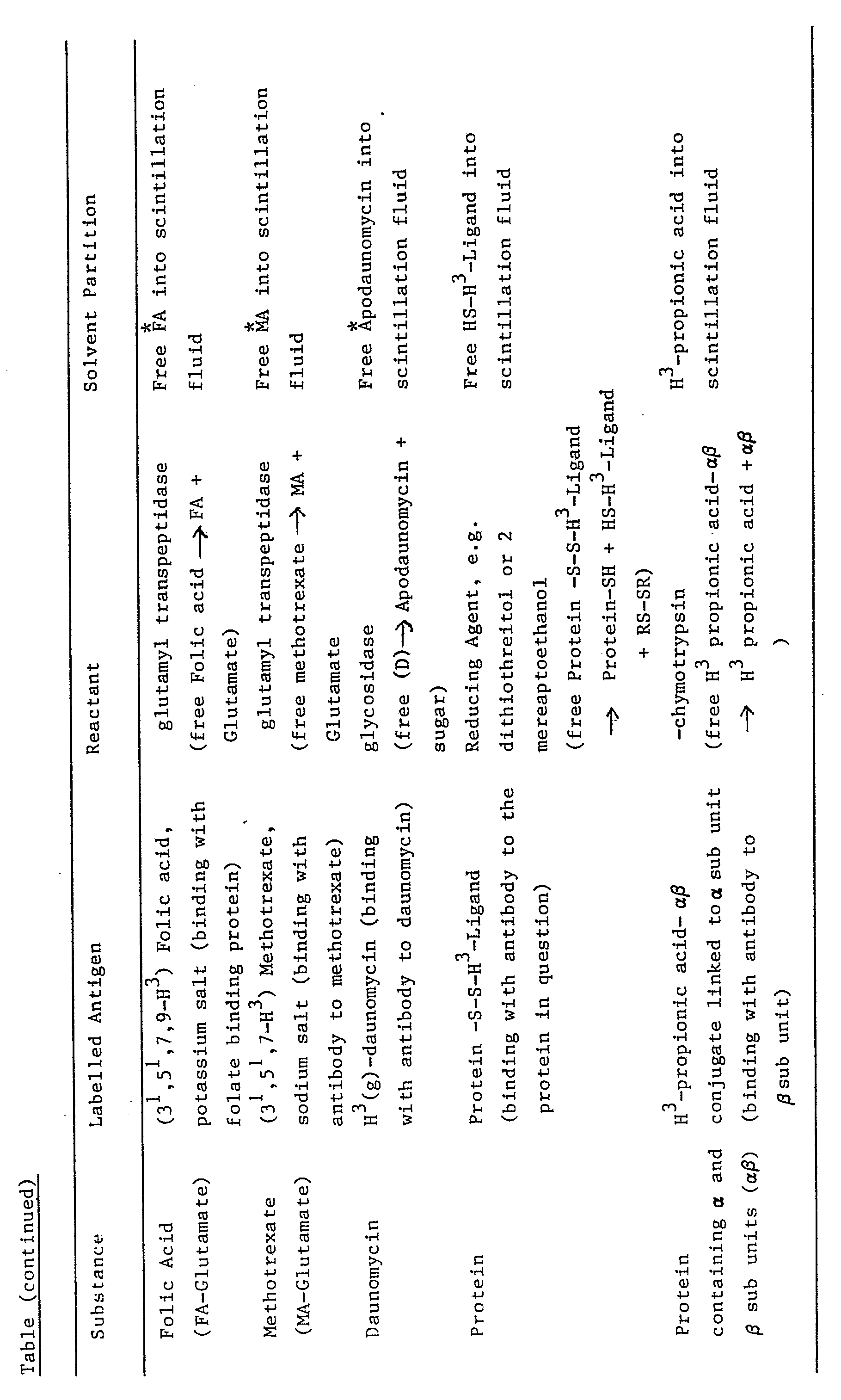

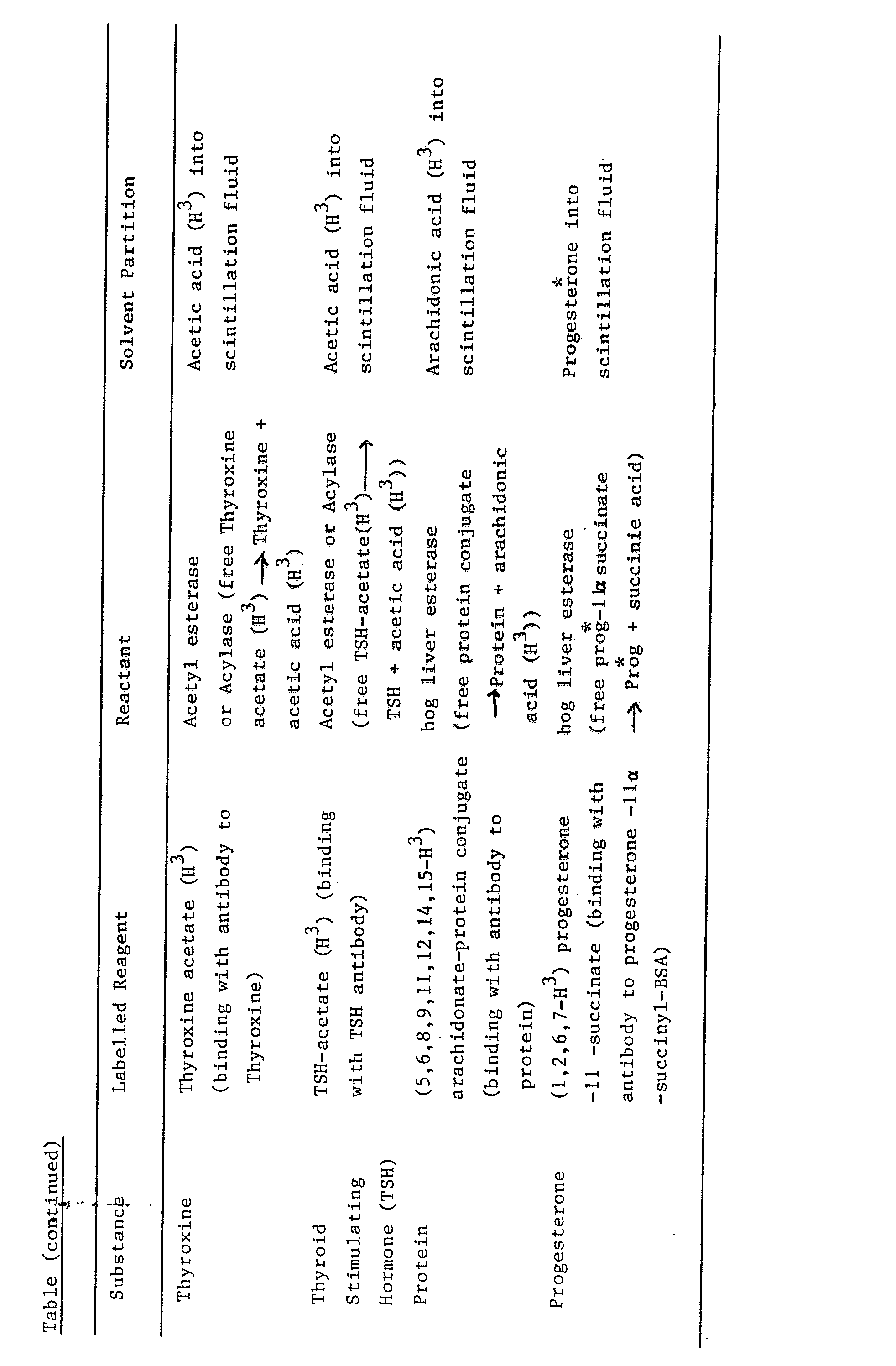

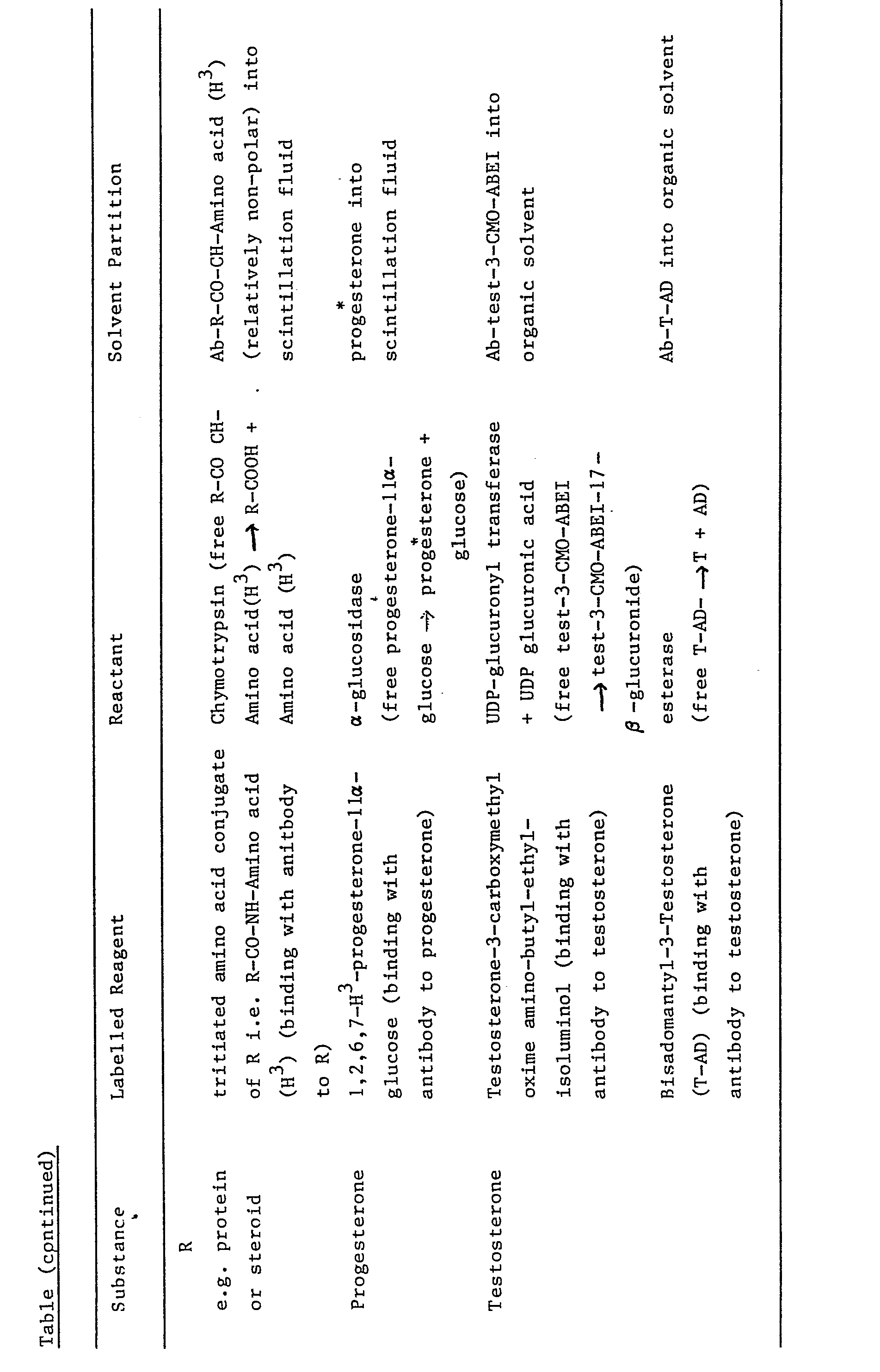

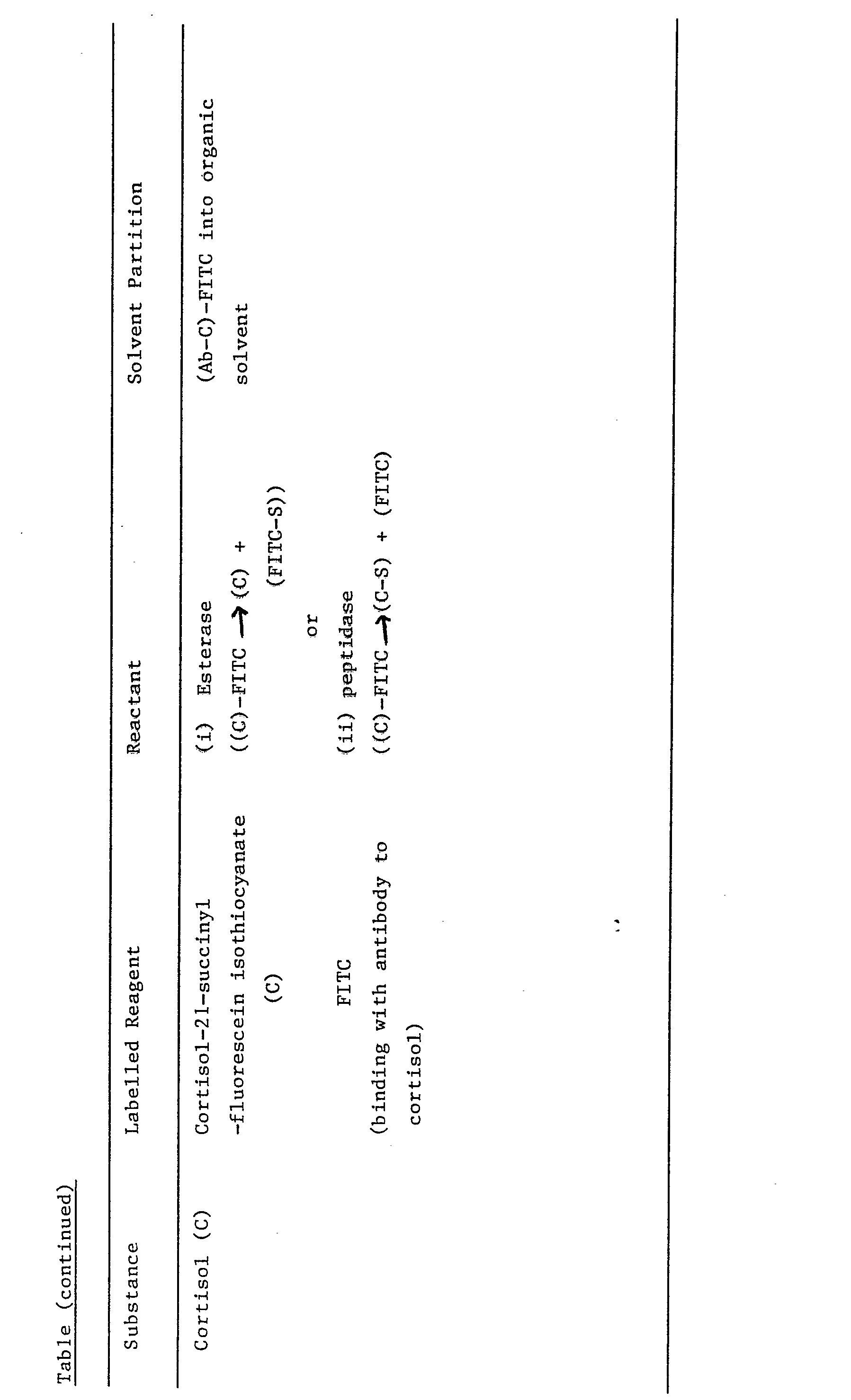

Other systems to which the method of the invention has been applied are described to further illustrate, through not limit the scope of, the invention in the following examples which refer to the accompanying diagrams in which:-
Figure 1 shows a graph of a standard curve of counts against oestradiol concentration;
Figure 2 shows a graph of the variation of plasma oestradiol in daily samples throughout a complete menstrual cycle as determined by LIDIA and conventional RIA;
Figure 3 shows a graph of the correlation between the concentration of oestradiol as determined by conventional RIA and the method of the invention (LIDIA);
Figure 4 shows a graph of a standard calibration curve for testosterone determination;
Figure 5 shows a graph of the correlation between concentration of testosterone as determined by a LIDIA method and a coventional RIA method, (a) for women and (b) for men;
Figure 6 shows a graph of a standard curve of counts against oestrone-3-glucuronide (Oeₗ-3-G) concentration;
Figure 7 shows a graph of the concentration variation of oestrone-3-glucuronide (Oeₗ-3-G) in daily samples of early morning urine throughout a complete menstrual cycle as measured by the method of the invention (LIDIA) and compared with a conventional radioimmunoassay method;
Figure 8 shows a graph of the correlation between the concentration of oestrone-3-glucuronide as determined by conventional RIA and LIDIA.
Figure 9 shows a graph of a calibration curve for thyroxine determination;
Figure 10 shows a correlation graph for thyroxine concentration as determined by conventional RIA and LIDIA;
Figure 11 shows a graph of a calibration curve for PGFM determination;
Figure 12 shows a graph of a calibration curve for digoxin determination;
Figure 13 shows a correlation graph of digoxin concentration determined by conventional RIA and LIDIA;
Figure 14 shows a diagrammatic representation of an automated continuous flow system for LIDIA, and
Figure 15 shows a graph of the concentration variation of oestriol-16a-glucuronide in serial samples of early morning urine throughout a complete menstrual cycle determined by the LIDIA method and compared with results obtained by conventional RIA.

### Example 1

### The determination of gonadal steroids in peripheral plasma

### (a) The determination of oestradiol

Plasma samples were taken daily throughout a menstrual cycle and the oestradiol (E ) content was determined by conventional RIA and also the method of the invention, (LIDIA).

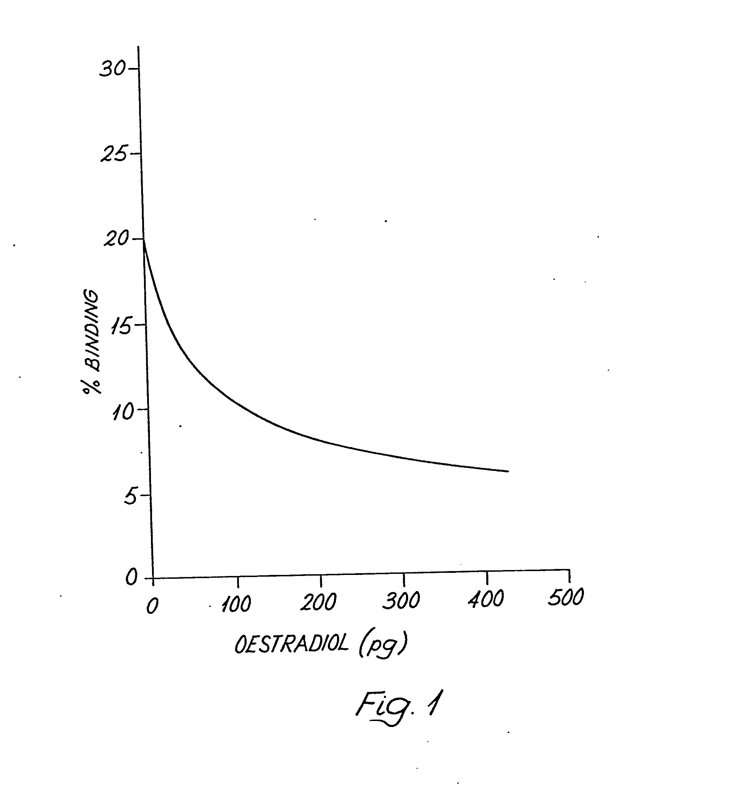

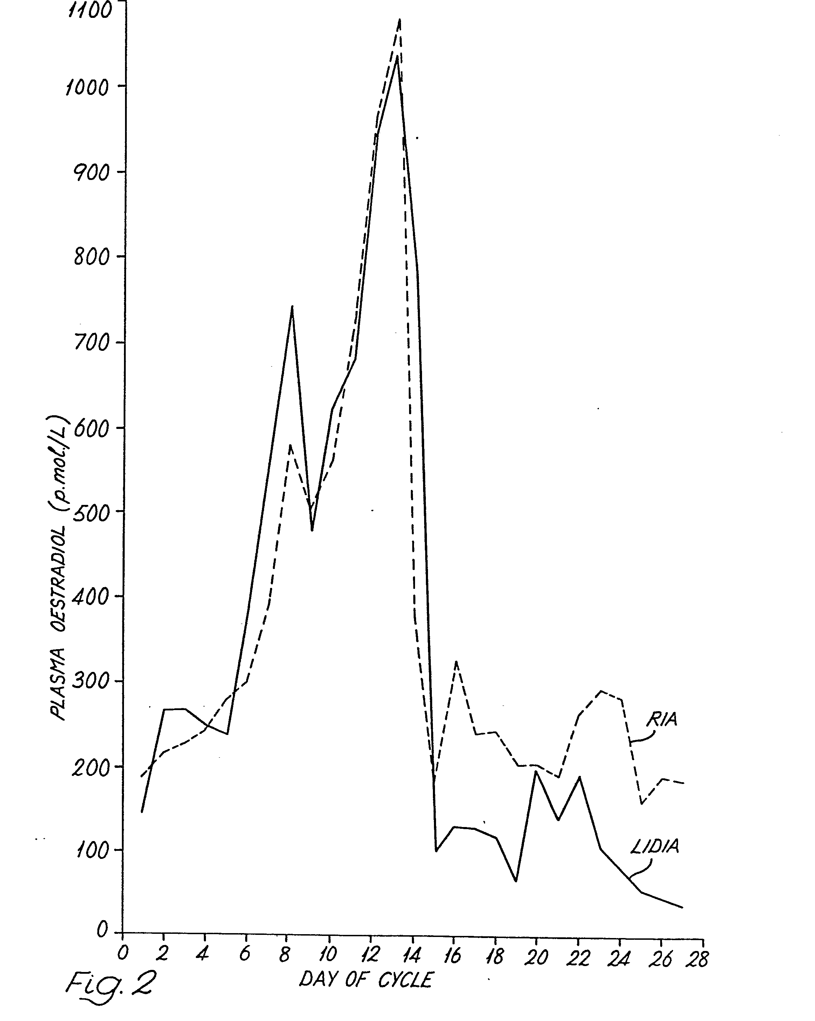

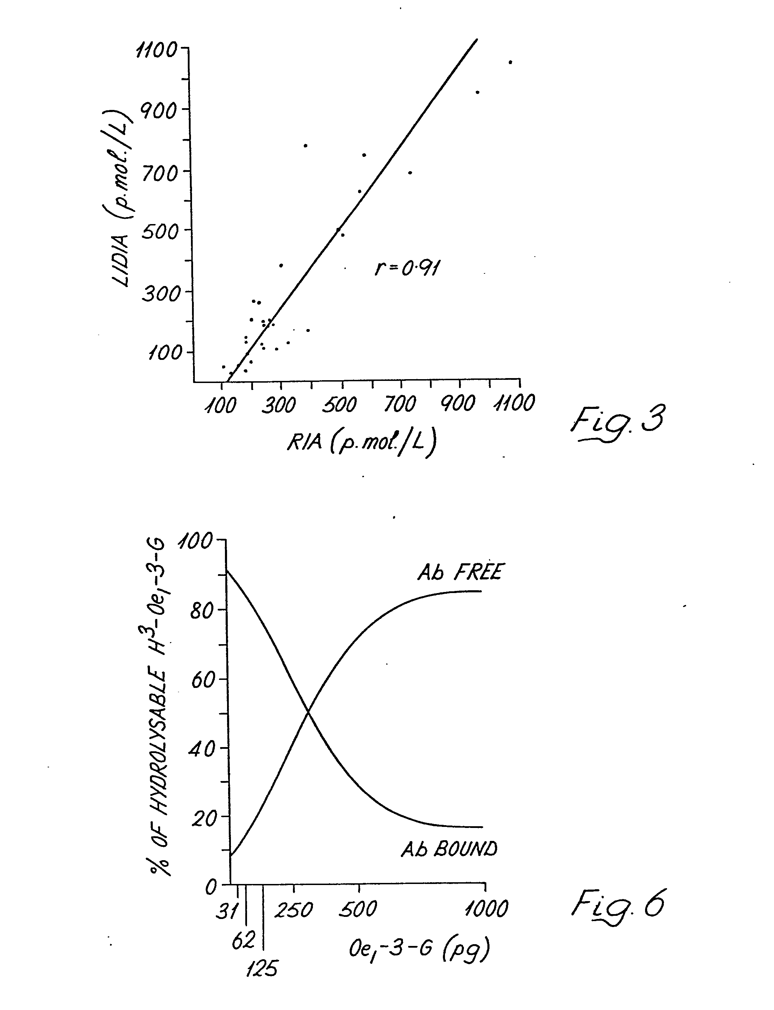

In the latter determination the method which was adopted was as follows: 200µl samples of plasma were extracted with 1 ml aliquots of diethyl ether in duplicate. The aqueous fractions were frozen with dry ice and ethanol, and the organic phases were decanted into assay tubes and evaporated to dryness in a vacuum oven. Labelled antigen (tritiated oestradiol; 50µl = 20,000 dpm) and antibody (anti-oestradiol-6-carboxymethyl oxime-BSA; 50P1 1:1,000 v/v) was added to each of the assay tubes, and the contents mixed and incubated at room temperature for 30 minutes. Aliquots of UDP-glucuronyl transferase (50µl=50µg) together with UDP glucuronic acid (50µl≡50µg) were then added to each tube and the mixture incubated at room temperature for 15 minutes, after which 1 ml of scintillation fluid (5g PPO/1 toluene:ethanol) was added, with mixing, to each assay tube and the radioactive counts determined in a scintillation counter. A standard curve, as shown in Figure 1, of radioactive counts against oestradiol concentration was also obtained using 50P1 samples containing 500, 250, 125, 62.5, 31.25 and 15.625 pg of oestradiol. The amounts of oestradiol present in the unknown samples were determined by comparison with the standard curve. The results obtained are given in Figure 2 which shows the variation in daily count as determined by the method of the invention compared with the results obtained by conventional RIA. Figure 3 shows the correlation between the concentration of oestradiol as determined by conventional RIA and the method of the invention. The method of the invention gives results which are clearly in excellent agreement with those obtained by a prior art RIA method.

Free labelled hormone present in the reaction mixture after the binding reaction interacts with the UDP-glucuronyl transferase conjugating enzyme in the presence of the UDP-glucuronic acid co-factor to produce labelled water soluble oestradiol -3(1-^{p})-glucuronide conjugate. On addition of the hydrophobic scintillant fluid, the water soluble conjugates are trapped in the aqueous phase, but hydrophobic labelled hormone derived from the hormone- antibody complex is released therefrom, and passes into the hydrophobic scintillant phase where it is determined.

### (b) Determination of oestrone

The oestrone content of peripheral plasma was determined by a LIDIA method similar to that described above for determination of oestradiol. 2, 4, 6, 7 - H³⁻oestrone labelled antigen (specific activity 85Ci/m mol) and antiserum to oestrone-6-carboxymethyl oxime-bovine serum albumin were used. Otherwise the reagents i.e. enzyme/UDPGA substrate and solvent/scintillant, and method were as for the determination of oestradiol. A calibration curve similar to that for a conventional heterogeneous radioimmunoassay was obtained by the LIDIA method.

### (c) Determination of oestriol

Similarly, as above, the oestriol content of peripheral plasma was determined using a LIDIA method. The labelled antigen used was 2, 4, 6, 7 - H³ oestriol (specific activity 100 Ci/m mol) and the antiserum was antiserum to oestriol-6-carboxymethyl oxime-bovine serum albumin. Otherwise the reagents and method were as for oestradiol.

A similar calibration curve to that obtained by conventional, heterogeneous RIA was obtained using the LIDIA method for oestriol.

### (d) Determination of testosterone

Testosterone concentration was determined in peripheral plasma of 13 women who were attending an infertility clinic and also in the plasma of 13 healthy men by a LIDIA method according to the present invention. For the sake of comparison the testosterone content of the same 26 samples was also determined by a conventional radioimmunoassay method.

The LIDIA method employed for testosterone determination was as follows:-

50µl samples of plasma from the women, or 20pl samples of plasma from the men, were extracted with 500pl aliquots of diethyl ether (in duplicate). The aqueous fractions were separated by freezing with dry ice and ethanol, and the organic phases were decanted into assay tubes and evacuated to dryness in a vacuum oven. Labelled antigen, tritiated testosterone (1, 2, 6, 7 - _{H}³⁻ testosterone - specific activity 93Ci/m mol; 100µ1≡20,000 dpm), together with antiserum (50µl anti testosterone-3-carboxymethyl oxime-bovine serum albumin) was added to each assay tube, mixed and then incubated at room temperature for 45 minutes. Aliquots of UDP-glucuronyl transferase (20µl=400µg protein), derived as a microsomal preparation from female hamster liver, together with UDP glucuronic acid were then added to each tube and the resultant mixtures incubated at room temperature for 90 minutes, after which 1 ml of scintillation fluid. (5g PPO/1 toluene:ethanol 991,v/v) was added, with mixing, to each assay tube and the radioactive counts determined in a scintillation counter.

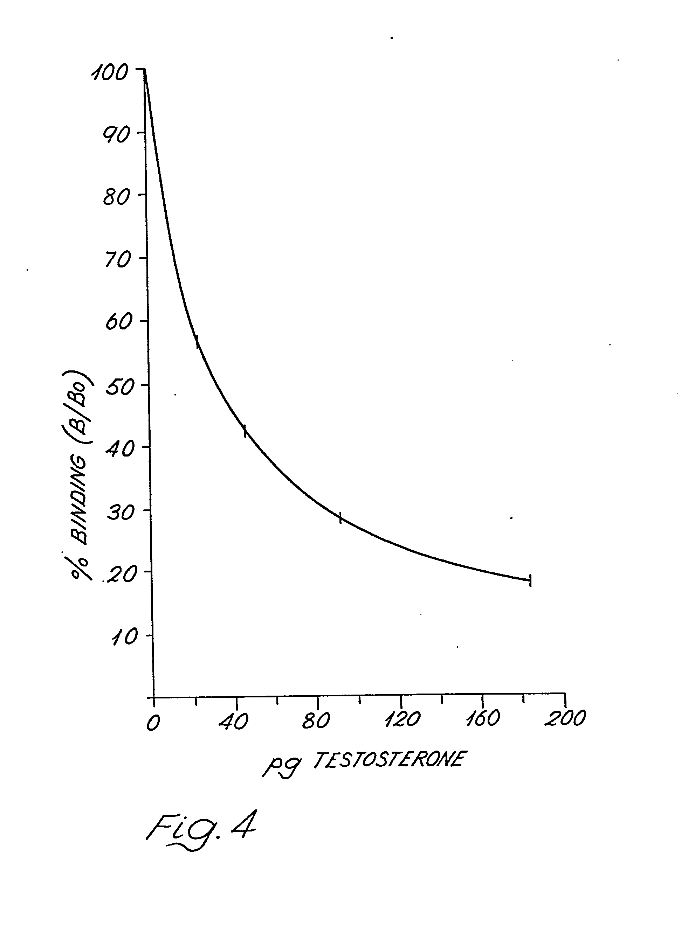

A standard calibration curve, as shown in Figure 4, of percentage binding.(i.e. (counts-enzyme blank/Bo-enzyme blsnk) x 100, where Bo are the counts at zero binding and zero testosterone concentration) versus concentration of testosterone, was obtained using 100µl samples of buffer containing 184, 92, 46, 23, 11.5, 5.75 and 2.87 pg of testosterone.

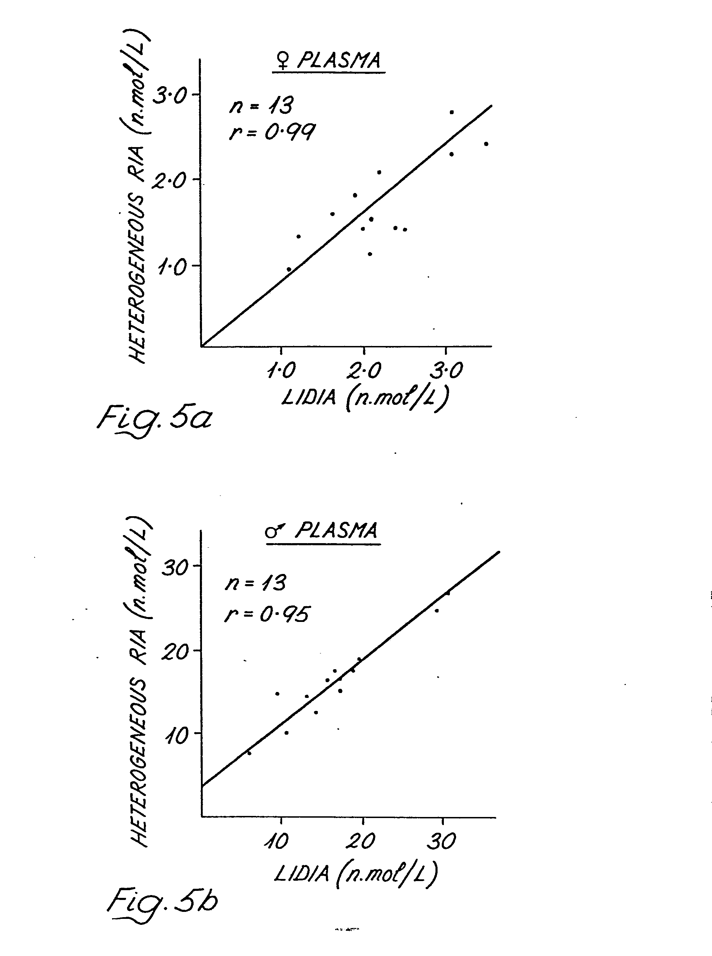

The testosterone content of the 26 patient samples were determined by comparison with the standard calibration curve and the results obtained by the LIDIA method were found to have a similar sensitivity to those obtained by the conventional, heterogeneous RIA method. A comparison of the results obtained by the two methods is given in Figure 5 which is in the form of correlation graphs (a) for women and (b) for men showing excellent agreement between the results obtained by RIA and LIDIA.

### Example 2

### The determination of water soluble gonadal steroid conjugates

### (a) Determination of oestrone-3-glucuronide

Oestrone-3-glucuronide (Oeₗ-3-G) was determined daily in samples of early morning urine throughout a complete menstrual cycle by conventional RIA and also by the method of the invention.

In the latter determination the method which was adopted was as follows: samples of urine were diluted 1:20 (v/v) with 0.01M phosphate buffer (pH 7.0). 50µl aliquots of the diluted sample were added to assay tubes together with 50µl of tritiated 0e₁-3-G in buffer (6, 7(n)-H³-E₁-3-G, specific activity 38Ci/m mol; 50µl =20,000 dpm) and 50µl of anti-Oeₗ-3-G (50µl=1:3,000 v/v). The contents of the tubes were mixed and incubated at 37⁰C for 15 minutes, 50pl (5U) of beta-glucuronidase (Type IX - Sigma Chemical Co. Ltd.) was then added to each tube and the contents mixed and incubated at 37⁰C for 10 minutes. 1 ml of scintillation fluid (3g PPO/1 toluene: ethanol; 99:1 v/v)was added to each tube, mixed and the radioactivity determined in a scintillation counter. A standard curve, as shown in Figure 6, of radioactive counts against oestrone-3-glucuronide concentration was obtained using 50µl samples containing 1,000; 500; 250; 125; 62 and 31 pg of oestrone-3-glucuronide. The concentration of oestrone-3-glucuronide present in the unknown samples was determined from the counts obtained by comparison with the standard curve. The results obtained are given in Figures 7 and 8, Figure 7 showing the daily concentration variation of oestrone-3-glucuronide in samples of early morning urine as measured by the method of the present invention (LIDIA) and also by conventional RIA, and Figure 8 showing the correlation between the concentrations of oestrone-3- glucuronide as determined by conventional RIA and the method of the invention. There was excellent agreement between the results obtained by the method of the invention and the conventional RIA method.

Also the sensitivity, precision and bias of the method was evaluated and compared with that of the conventional, heterogeneous immunoassay, the results obtained being given below in Table 1.

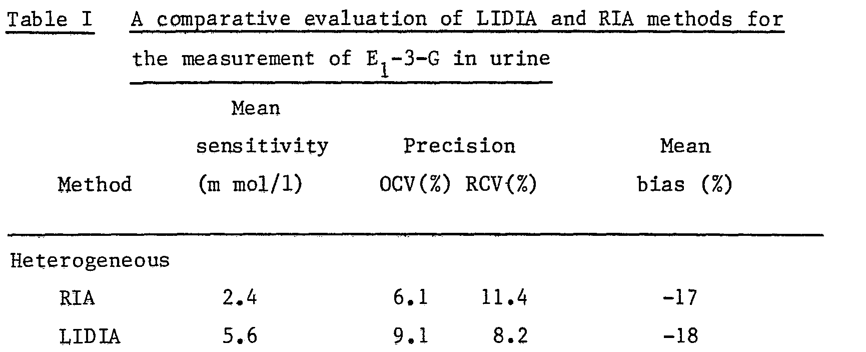

In Table I sensitivity relates to the concentration which was significantly different from zero on the standard curve (mean of 4) and the bias refers to the mean percentage difference of the results from a range of urine samples assayed before and after the addition of a known amount of metabolite (from 50 to 150 n mol/I). The precision was assessed by measuring the optimal conditions variance (OCV), i.e. the coefficient of variation over 20 analyses of the sample under ideal conditions, and the routine conditions variance (RCV), i.e. the coefficient of variation on the same urine sample analysed over 5 sequential assays. The quality control urine contained approximately 39.5 n mol/l of Eₗ-3-G.

Labelled oestrone-3-glucuronide remaining unbound in the reaction mixture subsequent to the binding reaction is hydrolysed by the enzyme to produce hydrophobic labelled oestrone which is then partitioned into the hydrophobic scintillant phase where it is determined.

### (b) Determination of oestriol -16a-glucuronide

Oestriol-16ct-glucuronide (E₃-16α-G) was determined in diluted urine of pregnant and non-pregnant women by a LIDIA method similar to that described above for determination of oestrone-3-glucuronide. The labelled antigen used was 6,9-H³-E₃-16α-G (specific activity 22Ci/m mol), and the antibody anti E₃-16 -G-6-BSA. Otherwise the reagents, enzyme and scintillant, and method were as for oestrone-3-glucuronide determination. The sensitivity, precision and values determined throughout a menstrual cycle were similar to those obtained by a conventional, heterogeneous radioimmunoassay.

### (c) Determination of pregnanediol -3a-glucuronide

The content of pregnanediol-3α-glucuronide (Pd-3a-G) in diluted urine was determined throughout a menstrual cycle by a LIDIA method similar to that described above for determination of oestrone-3-glucuronide.

Urine samples were diluted 1:100 (v/v) with 0.01M phosphate buffer (pH 7.0), and 50µl aliquots of the diluted samples were added to assay tubes together with labelled antigen (6,7-H³-Pd-3α-G, specific activity 42Ci/m mol; 50µl=20,000 dpm) and antibody in buffer (anti Pd-3x-G-6-BSA; 50µl of 1:2,000 v/v, i.e. 50% binding), mixed and incubated at 37⁰C for 15 minutes. 50µl (=5U) of β-glucuronidase (Type IX - Sigma Chemical Co. Ltd.) was added to each tube, the contents mixed and incubated at 37⁰C for 10 minutes; after which 1 ml of scintillation fluid (3g PPO/litre toluene: ethanol; 99:1 v/v) was added to each tube with mixing and the tritium content of the scintillant layers determined using a suitable scintillation counter. Standard tubes containing 10,000, 5,000, 2,500, 1,250, 625, 312 and 156 pg of Pd-3α-G/50µl buffer were also assayed by the above method, and a standard calibration curve was prepared from the results, being similar to the standard curve obtained by conventional, heterogenous RIA. The Pd-3a⁻G content of the unknown samples was determined by comparison with the standard curve. The sensivitiy of the LIDIA method was found to be similar to that of a conventional, heterogeneous RIA.

### (d) Determination of pregnanetriol-3a-glucuronide

The pregnanetriol-3α-glucuronide (Pt-3a-G) content of diluted urine from both men and women was determined by a LIDIA method similar to the methods described previously for determination of other conjugated steroids. Urine samples were diluted 1:50(v/v) with 0.01M phosphate and 50pl aliquots were added to assay tubes together with labelled antigen in buffer (50µl=20,000 dpm, 50% binding, 6,7(n)-H³-Pt-3α-G, specific activity 22Ci/mol) and antiserum in buffer (50µl of 1;2,000v/v anti Pt-3a-G-6-BSA). Otherwise the method and reagents used were as for the determination of oestrone-3-glucuronide.

The standard calibration curve obtained and the sensivity of the method were similar to that of a conventional, heterogeneous RIA for Pₜ-3a-G.

### (e) Determination of testosterone-17β-glucuronide

The testosterone-17p-glucuronide (T-17p-G) content of urine from both men and women was determined by a LIDIA method similar to that described above for determination of steroid conjugates. Urine samples were diluted 1:10(v/v) for females and 1:100(v/v) for males with phosphate buffer. 50µl aliquots of the diluted urines were used for assay being mixed with 50pl aliquots in buffer of labelled antigen (1,2(n)-H³-T-17β-G, specific activity 57Ci/mmol-20,000 cpm/50pl) and antigen (anti T-17p-G-6-bovine thyroglobulin; 50µl of 1:2,000v/v to give 50% binding). Otherwise the method and reagents used were as for the determination of oestrone-3-glucuronide. A calibration curve was obtained using standards containing 160, 80, 40, 20, 10 and 5 pg/50pl of T-17β-G.

It was found that the calibration curve obtained and the sensitivity of the method were similar to that obtained by a conventional, heterogeneous RIA for T-17β-G.

### (f) Determination of oestrone-3-sulphate

The concentration of a steroid sulphate, oestrone-3-sulphate (E,-3-S), was also determined using a LIDIA method similar to that described previously for determination of other steroid conjugates. Samples were diluted (e.g. urine samples) or extracted (e.g. serum samples) and mixed with 100µl in buffer aliquots of labelled antigen (6,7(n)-H³E₁,-3-S, specific activity l.lCi/mol; 20,000 dpm=100µl buffer) and antibody (anti Eₗ-3-S-6-BSA; 100µ of 1:500 v/v) in sample tubes and incubated at room temperature for 30 minutes. 100pl of enzyme, aryl sulphatase (Helix pomatia juice, Sigma Chemical Co. Ltd.; 100µl=10U) was then added to each tube, the contents mixed and incubated at room temperature for 30 minutes, after which 1 ml of scintillation fluid (3g PPO/litre, toluene: ethanol 99:lv/v) was added to each tube and the tritium content of the scintillant phase determined using a suitable scintillation counter. A standard calibration curve was also obtained from sample tubes containing 5,000, 2,500, 1,250, 625, 312, 156 and 76µpg/100µl of buffer of E₁-3-S and was found to be similar to that obtained by a conventional, heterogeneous RIA. The oestrone sulphate concentration in the unknown samples was determined by comparison with the standard calibration curve, and the results obtained indicated that the LIDIA method was as sensitive as a conventional RIA.

### Example 3 Determination of thyroid hormones

A LIDIA assay was developed for determination of total serum thyroxine (3,3',5,5'-tetraiodothyronine; T ) for use as a preliminary step in the assessment of thyroid function. The LIDIA assay was based on the use of a labelled propionic acid derivative of T₄ which competes for antibody with the natural T₄ present in the sample. The T₄ derivative was found to be susceptible to cleavage by an enzyme when in the free state, but not when in the bound state, releasing labelled propionic acid which was determined as a component of a hydrophobic solvent phase. The principle upon which the assay was based is as follows in schematic form:-(i) Competitive binding reaction

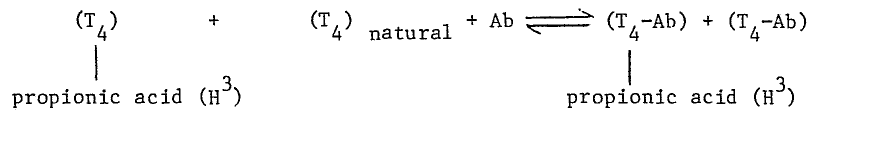

### (ii) Differential antigen metabolism

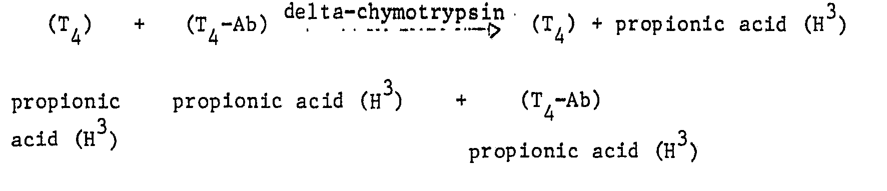

### (iii) Solvent partition

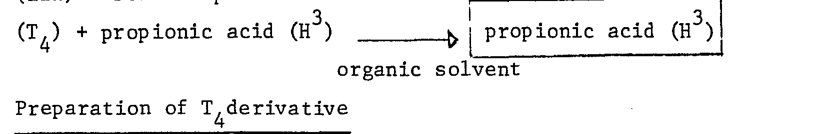

The tritium labelled propionic acid derivative of T₄ was prepared as follows:-

N-succinimidyl (2,3-H³) propionate (specific activity 50Ci/m mol, The Radiochemical Centre, Amersham, Bucks.) was used as acylating agent, reacting in an analogous manner to the Bolton & Hunter reagent. A quantity of the acylating agent (500µ Ci; 12 n mol) in toluene (250µl) was dried under a pressure of 0 mm Hg and cooled to 4°C. 3.1 mg of L-thyroxine was dissolved in 750µl of ethanol and 2.35 ml of distilled water was added. 5pl of the resultant solution (i.e. equivalent to 6.4 n mol of T₄) was then added to the dried ester and the resultant mixture incubated for 3 hours at 4°C, after which the reaction was stopped by addition of and mixing with 200µl of borate buffer (O.lM, pH 8.5). Unreacted, hydrolysed labelled propionic acid remaining in the reaction mixture was removed by extraction with a mixture of decanol and toluene (30:70; v/v).

### Assay

The procedure adopted for the assay was as follows:

Serum samples were diluted 1 + 3 with buffer (0.1M PBS; pH 7.5), 50µl aliquots of the diluted samples being used in each assay tube. Standard solutions in hormone free plasma (diluted 1 + 3 with buffer) were also prepared continaing T₄in the range from 400 to 12.5 n mol/l, 50µl aliquots of the standard solutions being taken in duplicate for the assay. To each aliquot of sample or standard solution was added labelled T₄ analogue (50µl containing 10,000 dpm) and 100µg of ANS (anilonaphthalene sulphonic acid) to displace any T₄ bound to thyroglobulin and pre-albumin) and anti- thyroxine sera (from RAST Allergy Unit, Benenden, Kent, diluted to give approximately 20% binding). The contents of each assay tube were then mixed and incubated for 1 hour at room temperature, after which aliquots of delta-chymotrypsin (50pl=10U, Sigma Chemical Co. Ltd.) were added with mixing and the resultant mixtures incubated for a further 15 minutes at room temperature. 1.5 ml of scintillaton fluid (5g PPO/litre, decanol: toluene, 30:70v/v) was then added to each tube with mixing, and the tritium content of the scintillant phases determined in a suitable scintillation counter.

A calibration curve (B/F versus log concentration of T₄) was prepared from the results obtained from the standard solutions and is shown in Figure 9.

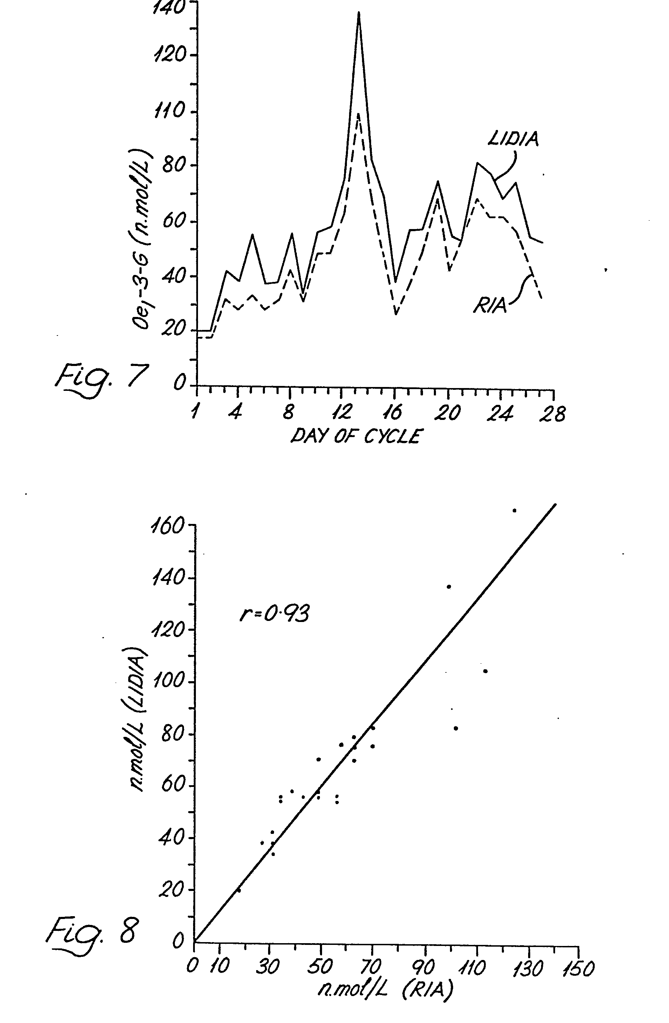

Seventeen serum samples which had been previously analysed for T₄ by a conventional, heterogeneous RIA were re-analysed by the LIDIA method described above. A comparison of the results obtained by the two methods is given in the form of a correlation graph in Figure 10.

### Example 4 Determination of prostanoids

A further LIDIA assay was developed for determination of the prostanoid 13, 14-dihydro-15-oxo-prostaglandin F₂ a(PGFM), which is the main circulating metabolite of prostaglandin F₂ a. The assay used relied upon the effect of ammonium sulphate treatment on the solubility properties of PGFM. It has been found that treatment of PGFM with saturated ammonium sulphate solution renders the normally hydrophilic free form of the prostanoid hydrophobic and thus organic solvent soluble, and it is believed, without prejudice, that this is due to the ammonium sulphate reversing the normal ionisation of carboxylic acid groups on the PGFM.

The method of assay adopted was as follows:

Samples were prepared by adding 100pl N HC1 to a 1 ml serum sample and extracting with 10 ml of diethyl ether, after which the organic phase was separated, dried and the residue redissolved in 500µl of assay buffer, 100pl aliquots in duplicate of the resultant solution being used for assay. Standard solutions were also prepared containing 1,000, 500, 250, 125, 62.5, 31.25, 15.62 and 7.8 pg/100µl of the prostanoid in buffer, 100µl aliquots of the solutions being used for assay. To assay tubes containing the 100µl aliquots of sample or standard were added aliquots of labelled PGFM (5, 6, 8, 11, 12, 14-H³-PGFM; specific activity 75Ci/m mol, 100µl:20,000 dpm) and antisera (anti PGFM-1-bovine thyroglobulin), the contents mixed and incubated for 20 minutes at room temperature. 300µl of saturated aqueous ammonium sulphate solution was then added, with mixing to each tube, after which 1 ml of scintillation fluid (5g PPO/litre toluene:ethanol, 99:1 v/v) was added with mixing. The tritium content of the scintillant layer of each tube was then determined using a scintillation counter.

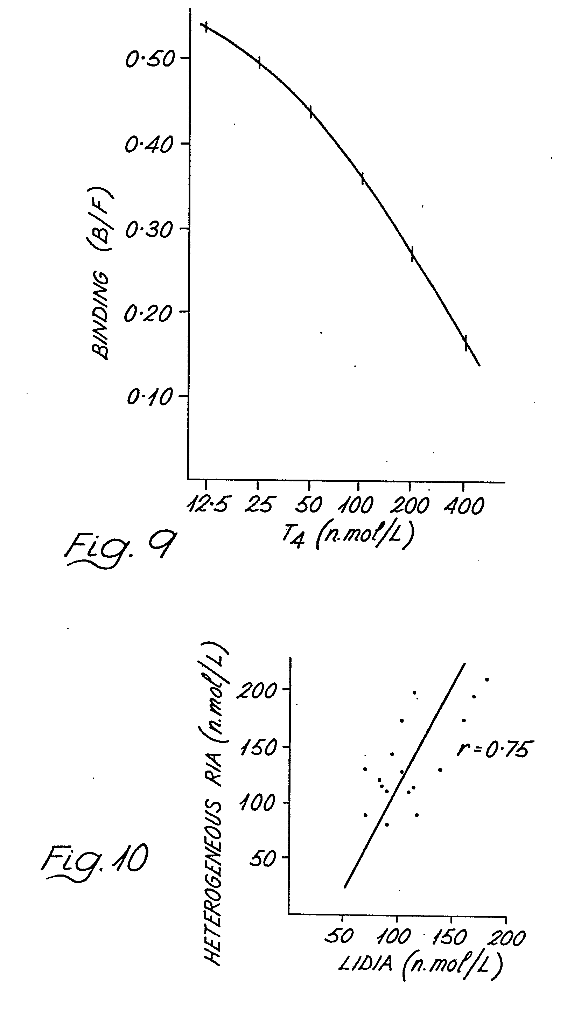

A calibration curve (% FREE/TOTAL versus PGFM concentration in pg/tube) was prepared from the results obtained from the standard solutions and is shown in Figure 11. The LIDIA method was found to have similar sensitivity and precision to that of a conventional, heterageneous RIA.

### Example 5 Determination of drugs

A LIDIA assay was also developed for determination of the drug digoxin. This assay, similar to the LIDIA assay for PGFM described in Example 4, relied upon ammonium sulphate treatment to render the normally hydrophilic form of the drug hydrophobic and organic solvent-soluble, and thereby facilitate partition of free labelled drug material into an organic phase away from complexed labelled drug material present in the aqueous phase.

The method employed for the assay was as follows:

50µl plasma samples were made up in duplicate in sample tubes. To each tube was added aliquots of labelled digoxin (12aH^{3_}Digoxin, specific activity 14Ci/m mol; 50µl=10,000 dpm) and antiserum to digoxin (50pl), and the contents of the tubes were mixed and incubated at 4⁰C for 30 minutes. 300µl of saturated aqueous ammonium sulphate solution were then added to each tube with mixing, followed by 1 ml of scintillation fluid (5g PPO/litre toluene: ethanol, 99:lv/v) with mixing, after which the tritium content of the scintillant phases were determined using a suitable scintillation counter. Standard solutions in stripped plasma were also prepared and assayed, containing 5,000, 2,500, 1,250, 625, 312, 156 and 78 pg of digoxin/50u1 buffer.

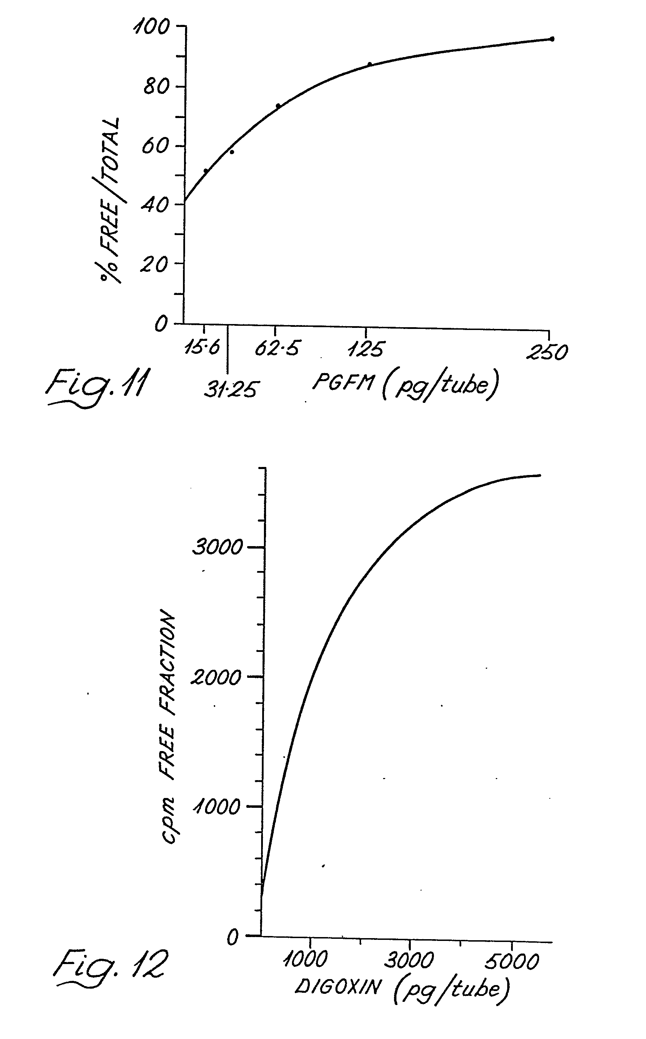

A calibration curve (counts per minute for the free fraction versus digoxin concentration in pg/tube) was prepared from the results obtained from the standard solutions and is shown in figure 12.

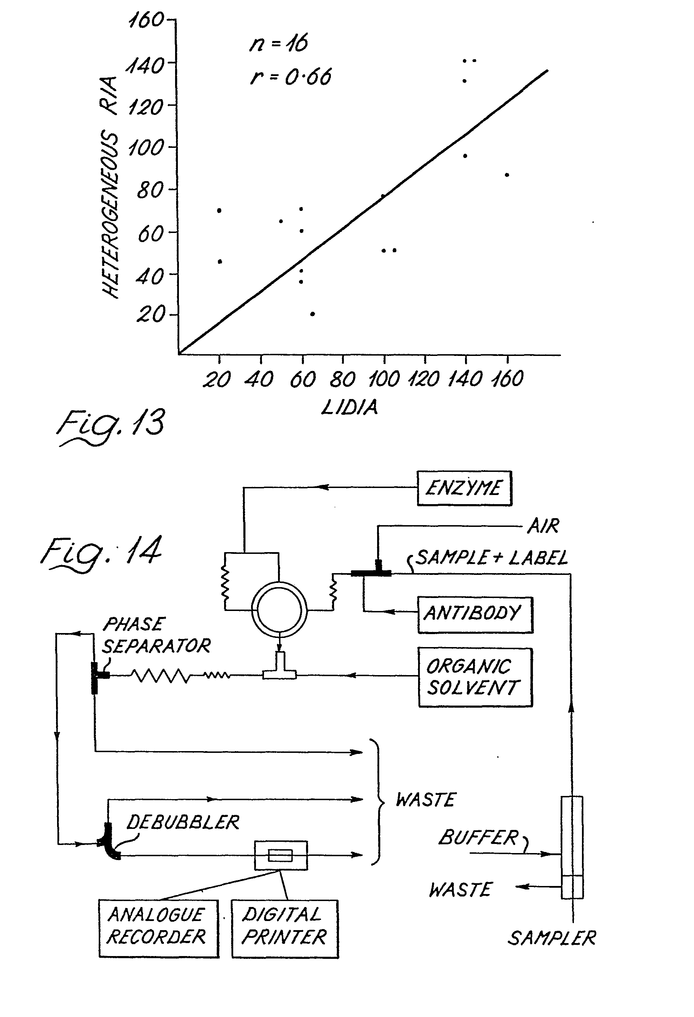

18 plasma samples from patients receiving digoxin treatment were analysised by the method described above. These samples had also been analysed previously by a conventional, heterogeneous RIA method. A comparison of the results obtained by the two methods is given in the form of a correlation graph in Figure 13.

### Example 6 Automation of LIDIA

An automated continuous flow analysis system, shown diagrammatically in Figure 14, was developed for use with the LIDIA method of the present invention. All reagents used, (i.e. sample and label, antibody, enzyme and organic solvent) are in liquid form and are added, mixed and incubated at appropriate points and mixing and incubation coils provided within the system. After addition of the organic solvent (e.g. scintillation fluid) a phase separator is employed to separate out the organic phase which is then passed to an appropriate detector via a debubbler.

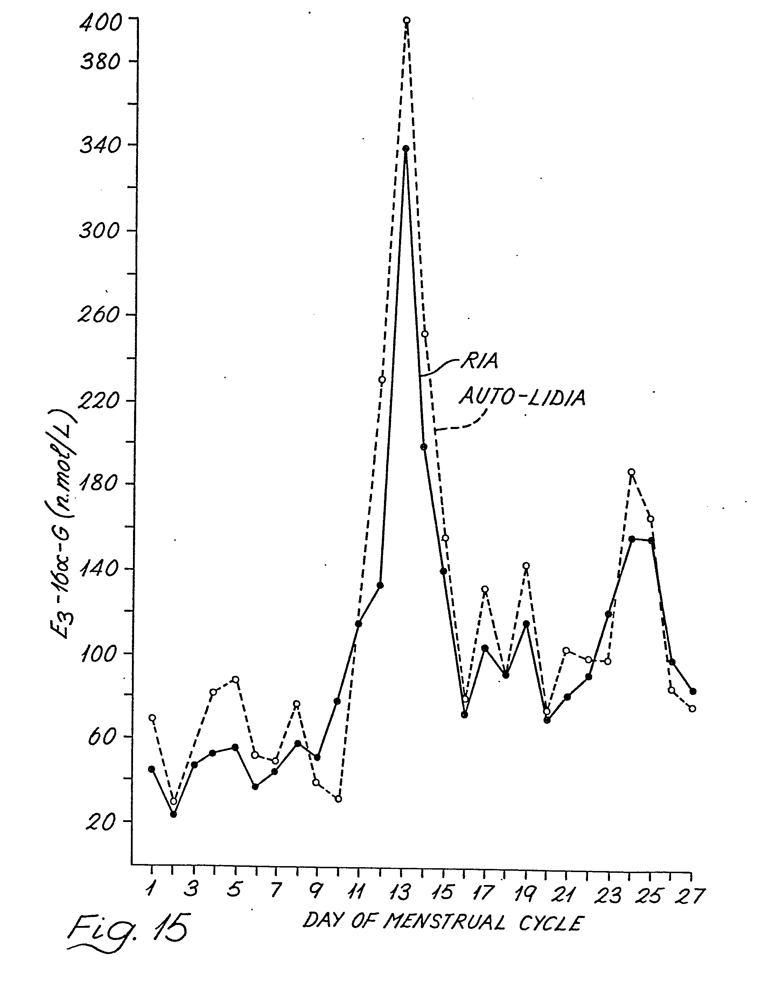

An automated system based on existing AAII technology and incorporating a liquid scintillation flow cell as the detector, was evaluated for the measurement of oestriol-16a-glucuronide. The reagents and principle of the method used were substantially as previously described in Example 2(b). The system was used to determine oestriol-16a-glucuronide content of serial samples of early morning urine throughout a complete menstrual cycle. The results obtained are given in Figure 15 which, for the sake of comparison, also contains results obtained by a conventional, heterogeneous, manual RIA analysis of the same samples. The results obtained by the automated LIDIA method compare favourably with those obtained by the conventional manual RIA method.

It will be appreciated that the automated LIDIA method described above may be applied equally to determination of other compounds besides oestriol-16a-glucuronide, and also to other methods using non-isotopic end points, such as fluorescent and luminescent end points.

### Example 7 LIDIA with non-isotopic labels

### (a) Oestradiol (^{E}₂)

Oestradiol-3-acridine ester was investigated for use as a reagent in a luminescent LIDIA assay for oestradiol. In such an assay the luminescent acridine ester competes with natural oestradiol present in the sample for a limited quantity of antibody, and acridine is preferentially cleaved from the free form of the ester by a suitable esterase or chemical agent, e.g. hog liver esterase or alkali, to give non-luminescent free acridine. Complexed acridine-oestradiol ester may then be partitioned from the reaction mixture using a suitable polar organic solvent and luminescence produced and determined in the organic solvent phase by oxidation of the acridine ester e.g. by potassium t-butoxide/DMSO. The method may be readily automated using a flow cell luminometer.

Preliminary investigations of this method have indicated that:
(i) chemiluminescence can be performed in an organic phase;
(ii) the labelled antigen can be cleaved enzymatically or chemically to release free acridine, and
(iii) free acridine is not luminescent.

Present results indicate, however, that the labelled antigen does not bind effectively to the antibodies, though it is anticipated that satisfactory labelled antigen - antibody binding can be obtained if appropriate derivatives to oestradiol-3-acridine ester are synthesised.

### (b) Testosterone (T)

A similar luminsecent LIDIA assay for testosterone was investigated based upon the use of testosterone-3-carboxymethyl oxime-amino butyl-ethyl-isoluminol (T-3-CMO-ABEI) (supplied by Dr. F. Kohen, The Weizmann Institute of Science, Israel); the method relying upon preferential cleavage of the labelled antigen, when in the free state, and partition of the labelled antigen, derived from complex, into a polar organic solvent phase, e.g. decanol.

Preliminary results have indicated that:
(i) chemiluminescence can be performed in an organic phase;
(ii) antibody binding inhibits partition of the labelled antigen into an organic phase, and
(iii) excess testosterone in the presence of antibody and labelled antigen (T-ABEI) enhances light emitted from the organic phase.

It will be appreciated that this method has not yet been optimised, and it is anticipated that appropriate choice of solvent systems will considerably improve the partition of labelled antigen away from the cleaved isoluminol moiety.

### Example 8 Determination of protein

Preliminary investigations were carried out on two alternative LIDIA methods for determination of the protein, human chorionic gonadotrophin (hcg).

### (a) hCG (5-H3)-tryptophan

In one method a tritiated tryptophan-hCG conjugate was investigated for use as the labelled antigen. This conjugate was prepared as follows:-

1 mg of hCG (10,000 IU/mg; Calbiochem Ltd.) was dissolved in a 100pl of phosphate buffer (3 mM; pH 6.3). An ethanolic solution of (5-H³)-tryptophan (2.5 mCi - 125 n mol; The Radiochemical Centre, Amersham) was dried under vacuum (0 mm Hg) at 50 C, and 500µg of hcG (=12.5n mol) in 50pl of the previously prepared solution was added and the resultant mixture cooled to 4°C. 3 mg of EDAC (1-ethyl-3-(3-dimethylaminopropyl) carbodiimide-HCl) was then added to the reaction mixture which was left overnight at 4⁰C. The reactants were separated and labelled hCG purified by repeated chromatography on a 60 cm Sephacryl-200 column.

This labelled antigen was investigated for use in a LIDIA assay in which the labelled material competes with natural hCG for a limited quantity of antibody and an enzyme tryptophanase is used to cleave tryptophan present in the free hCG - tryptophan conjugate and release tritium labelled indole which is partitioned into a scintillation fluid phase in.which its tritium content is determined.

### (b) hCG - (2,3-H³) propionate

In an alternative method a tritiated propionic acid - hCG conjugate was investigated for use as the labelled antibody. This conjugate was prepared by a method similar to that previously described in Example 3 for preparation of T₄-(2,3-H³) propionate except that 400µCi of the ester was reacted with 10µg ( - 250 p mol) of hCG. The labelled antigen was purified by chromatography on a Sephadex G25 column.

The LIDIA method using this labelled antigen is substantially the same in principle to that described above using the tritiated tryptophan - hCG conjugate as labelled antigen, except that the enzyme used is beta-chymotrypsin which gives rise to the production of tritium-labelled propionic acid from the conjugate.

Preliminary investigation of these two methods indicate that:
(i) both hCG - (2,3-H³) propionate and hCG-(5-H³) tryptophan bind to hCG antibodies;
(ii) hCG - (2,3-H³) propionate can be displaced from hCG antibodies by unlabelled hCG, and
(iii) hCG - (2,3-H³) propionate is hydrolysed by β-chymotrypsin to release hCG and (2,3-H³) propionic acid.

## Claims

1. A method of carrying out a binding assay comprising treating the aqueous reaction mixture, subsequent to the binding interaction, with a reactant which interacts preferentially with a labelled version of one or other of the binding reagents when said reagent is either in the free state or bound in a complex, to give rise to a product having solubility properties which are substantially different from those of the original binding reagent or complex, thereafter partitioning hydrophobic substances from the treated aqueous reaction mixture into a hydrophobic solvent phase and detecting or determining labelled material present in the hydrophobic phase.

2. A method according to Claim 1 comprising an immunoassay.

3. A method according to Claim 1, for assay of a hormone, vitamin, pharmacological agent, drug or metabolite, or binding substance or receptor of any of these.

4. A method according to any of the preceding Claims for assay of a hyrophilic metabolite conjugate of a hormone or drug.

5. A method according to any of the preceding Claims, in which the labelling used comprises a radionuclide, luminescent or fluorescent label.

6. A method according to Claim 5, in which the labelling used comprises a #-emitting radionuclide label and the hydrophobic solvent phase comprises scintillation fluid.

7. A method according to any of the preceding Claims, in which the reactant interacts preferentially with the labelled version of one or other of the binding reagents when said reagent is in the free state.

8. A method according to any of the preceding Claims, in which the product formed on interactinn of the reactant with the reagent comprises a fragment of the original reagent cleaved therefrom by the action of the reactant.

9. A method according to any of Claims 1-7, in which the product formed on interaction of the reactant with the reagent comprises the original reagent altered by addition or removal of a functional grouping by means of which the hydrophobic/hydrophilic solubility properties of the reagent are changed.

10. A method of carrying out a binding assay for a hydrophilic substance in which, subsequent to the binding interreaction, the aqueous reaction mixture is treated with a reactant which interacts preferentially with a free labelled version of the hydrophilic substance to give rise to a labelled hydrophobic product which is thereafter partitioned, from the treated aqueous reaction mixture and determined.

11. A method ofcarrying out a binding assay for a hydrophobic substance in which, subsequent to the binding interaction, the aqueous reaction mixture is treated with a reactant which interacts preferentially with a free labelled version of the hydrophobic substance to give rise to a hydrophilic product, hydrophobic bound complex comprising labelled material is thereafter partitioned from the treated aqueous reaction mixture into a hydrophobic phase and said labelled material is then determined.

12. A method according to any of the preceding Claims, in which the reactant comprises an enzyme.

13. A method according to Claim 12, in which the reactant comprises an esterase.

14. A method according to Claim 12, in which the reactant comprises a specific conjugating enzyme together with a suitable derivative.

15. A method according to Claim 1 for assay of progesterone, morphine, tetrahydrocannabinol, ethynyl oestradiol, oestrone-3- glucuronide. glycocholic acid, folic acid, methotrexate, daunomycin, thyroxine, thyroid stimulating hormone, testosterone or cortisol.

16. A method according to Claim 1 for assay of a gonadal steroid.

17. A method according to Claim 1 for assay of a water soluble gonadal steroid conjugate.

18. A method according to Claim 1 for assay of a thyroid hormone.

19. A method according to Claim 1 for assay of a prostanoid.

₂0. A method according to Claim 1 for assay of digoxin.

21. A method according to Claim 1 for assay of human chorionic. gonadotrophin.

₂2. A kit for use in a method according to Claim 1 comprising binding assay reagents and a reactant for interacting with a labelled version of one or other of the binding reagents when said reagent is either in the free state or bound in a complex to give rise to a product having solubility properties which are substantially different from those of the original binding reagent or complex.

23. A kit according to Claim 22, in which the reactant comprises an esterase or specific conjugating enzyme and derivative.

24. A kit according to Claim 22 or 23 comprising hydrophobic solvent for partitioning hydrophobic material from the aqueous reaction mixture.
